(19) Europäisches Patentamt
European Patent Office
Office européen des brevets

(11) **EP 1 204 862 B1**

(12) **FASCICULE DE BREVET EUROPEEN**

(45) Date de publication et mention de la délivrance du brevet:
**10.07.2013 Bulletin 2013/28**

(21) Numéro de dépôt: **01907837.7**

(22) Date de dépôt: **16.02.2001**

(51) Int Cl.:
***G01N 27/447*** (2006.01)

(86) Numéro de dépôt international:
**PCT/FR2001/000479**

(87) Numéro de publication internationale:
**WO 2001/069229 (20.09.2001 Gazette 2001/38)**

(54) **METHODE DE SEPARATION, D'IDENTIFICATION ET DE QUANTIFICATION DES ISOPHOSPHATASES ALCALINES PAR ELECTROPHORESE**

VERFAHREN ZUR TRENNUNG, IDENTIFIZIERUNG UND MESSUNG VON ALKALISCHEN ISOPHOSPHATASEN DURCH ELEKTROPHORESE

METHOD FOR SEPARATING, IDENTIFYING AND QUANTIFYING ALKALINE ISOPHOSPHATASES BY ELECTROPHORESIS

(84) Etats contractants désignés:
**AT BE CH CY DE DK ES FI FR GB GR IE IT LI LU MC NL PT SE TR**

(30) Priorité: **10.03.2000 FR 0003142**

(43) Date de publication de la demande:
**15.05.2002 Bulletin 2002/20**

(73) Titulaire: **SEBIA**
**92130 Issy-Les-Moulineaux (FR)**

(72) Inventeur: **BELLON, Franck**
**F-91160 Longjumeau (FR)**

(74) Mandataire: **Desaix, Anne**
**Ernest Gutmann - Yves Plasseraud S.A.S.**
**3, rue Auber**
**75009 Paris (FR)**

(56) Documents cités:
**EP-A- 0 131 606       EP-A- 0 848 251**
**WO-A-99/45374       US-A- 5 667 654**
**US-A- 5 853 668**

• **V. VAN HOOF: "comparison of two commercially available systems for the electrophoretic separatiion of alkaline phosphatase isoenzymes" JOURNAL OF CHROMATOGRAPHY, vol. 646, no. 1, 1993, pages 235-243, XP000981324 nl**

**Description**

La phosphatase alcaline (EC 3.1.3.1.) (abréviation ALP), est une metalloenzyme consistant en un groupe d'isoenzymes, présentes dans différents tissus des organismes animaux et en particulier chez l'homme.

Les isoenzymes de la phosphatase alcaline présentent un intérêt dans des protocoles de diagnostic de différentes affections chez l'adulte ou chez l'enfant, et plusieurs méthodes ont donc été proposées pour la séparation et la mesure de ces isoenzymes. On peut répartir les isoenzymes de l'ALP en quatre classes : tissulaire non-spécifique (os, foie et rein), intestinale adulte, intestinale foetate, placentaire. Au sein d'une même classe, il peut exister certaines variantes, à savoir :

- hépatiques : hépatique 1 (H1), hépatique 2 (H2) ;
- ultrafast UF ;
- osseuse (Os) ;
- placentaires : placentaire 1 (P1), placentaire 2 (P2)
- intestinales : intestinale 1 (I1), intestinale 2 (12), intestinale 3 (I3) ;

Neuf fractions principales sont donc distinguées, qu'il convient de pouvoir séparer, identifier et quantifier, notamment en vue de leur recherche dans des pathologies hépatiques et biliaires ainsi que dans certaines maladies des os, incluant les tumeurs osseuses, ou dans la maladie de Paget.

On utilisera parfois dans la suite le terme « fraction » pour désigner une classe d'isoenzymes de l'ALP ou une variante particulière au sein d'une classe d'isoenzymes. Sur le support d'électrophorèse, une « fraction » correspond à une bande révélée après la migration.

L'analyse de routine la plus pratiquée sur les isoenzymes de la phosphatase alcaline consiste à mesurer l'activité enzymatique totale au moyen d'un substrat de cette enzyme, généralement le paranitrophenylphosphate. Néanmoins, cette méthode ne permet pas d'obtenir le taux des différentes isoenzymes.

La principale méthode séparative d'analyse de ces composés fait appel aux techniques électrophorétiques. Ainsi, l'isoélectrofocalisation est parfois utilisée et permet d'obtenir une séparation en 10 à 20 bandes en fonction de la technique mise en oeuvre. L'identification de toutes ces bandes est difficile ce qui rend l'interprétation clinique extrêmement délicate.

L'électrophorèse de zone permet de séparer convenablement les principales formes d'isophosphatases. Cependant, certaines fractions sont superposées, notamment les fractions Os, H1, P1 et de ce fait des traitements complémentaires doivent être effectués pour les séparer et les identifier. Ces traitements doivent être réalisés sur les échantillons biologiques testés avant leur dépôt sur le gel.

Ces traitements consistent par exemple en une dénaturation thermique, une incubation avec des inhibiteurs spécifiques tels que l'urée, les acides aminés..., une incubation enzymatique avec la neuraminidase, la ficine, la phospholipase C, une incubation avec des antisérums spécifiques, anti placentaires ou anti intestinaux. Différentes techniques de séparation actuellement pratiquées ont été abordées dans Van Hoof V.O., De Broe Marc, E., Clinical Laboratory Sciences, vol. 31, issue 3 1994, Interpretation and clinical significance of alkaline phosphatase isoenzyme patterns.

Une technique particulière est proposée dans le brevet US 5,264,098, qui décrit la séparation des isoenzymes de l'ALP, au moyen d'une réaction d'électrophorèse sur gel mettant en oeuvre un tampon de gel contenant au moins un détergent non ionique et un détergent anionique.

Les traitements disponibles pour l'identification et la quantification des isoenzymes de l'ALP présentent certains inconvénients pour l'analyse de routine. En plus de leurs coûts élevés ils peuvent d'une part, être longs et alourdir considérablement la manipulation, d'autre part, pour une détermination complète (de la totalité des isoenzymes), il est nécessaire de réaliser plusieurs traitements (2 à 3) pour un seul échantillon, ce qui limite le nombre d'échantillons qui peut être analysé simultanément sur un même gel.

D'autres traitements sont proposés, qui prévoient par exemple le traitement de l'échantillon, préalablement au dépôt de l'échantillon sur le gel d'électrophorèse. Dans ce cadre, l'action de la lectine WGA (wheat Germ Aglutinin) est particulièrement intéressante (cf. Sidney B. Rosalski, A. Ying Foo, Clinical Chemistry, 30/7, p.1182-1186, 1984, Two new methods for separating and quantifying bone and liver alkaline phosphatase isoenzyme in plasma, brevet EP 0131606 du 5/11/86). Le brevet EP0131606 décrit ainsi la détection différenciée de l'isoenzyme de l'ALP des os et du foie, comprenant le traitement de l'échantillon testé avec de la lectine, puis l'incubation du mélange obtenu, suivie de la séparation de l'ALP liée à la lectine, de la fraction contenant l'ALP libre, et de la détermination dans l'un des deux milieux ou dans les deux, de l'activité ALP. Selon un mode de réalisation particulier de ce brevet, la séparation des deux fractions (ALP liée à la lectine et ALP libre) est réalisée par électrophorèse.

A l'exception des formes intestinales, toutes les isophosphatases possèdent des acides sialiques et sont donc plus ou moins affectées par un traitement à la lectine WGA. La fraction osseuse étant la plus sialilée; c'est elle qui sera la plus affectée par ce traitement ce qui lui confère, dans des conditions appropriées, un retard de mobilité et de ce fait la conduit à précipiter dans une zone distincte par rapport à la zone de localisation de la fraction hépatique.

Afin de rendre la précipitation des isoenzymes de l'ALP plus sélective vis à vis de l'isoenzyme osseuse, certains auteurs ont utilisé des détergents comme par exemple le triton X 100 (Rosalki). Mais, malgré la présence de ces détergents, il subsiste des interactions résiduelles de la lectine WGA avec d'autres isophosphatases qui entraînent des coprécipitations de ces fractions avec la fraction osseuse.

Outre ce manque de spécificité, un autre inconvénient de cette technique est d'alourdir considérablement l'analyse.

La publication Rosalki S.B. et al précitée propose, alternativement, l'incorporation de lectine dans le tampon utilisé pour imprégner le , gel d'électrophorèse, préalablement à l'utilisation de ce gel. Ceci permet d'éliminer le traitement préalable de l'échantillon. Dans ce cas, la majorité de la fraction osseuse est précipitée à proximité du dépôt de l'échantillon. Toutes les autres isoenzymes, à l'exception des isoenzymes intestinales voient leur mobilité affectée par l'action de la lectine WGA et ce, malgré la présence des détergents susmentionnés.

Dans le cadre de ce traitement, les propriétés de la lectine mises en oeuvre, sont sa capacité à interagir spécifiquement avec les isoenzymes de l'ALP possédant des acides sialiques.

Le brevet US 5,667,654 décrit le dépôt d'un réactif après la séparation électrophorétique des constituants d'un échantillon et après que lesdits constituants séparés ont été maintenus dans un état statique particulier. Parmi les activités susceptibles d'être révélées par un réactif, figure l'activité phosphatase alcaline.

La présente invention propose des moyens pour surmonter, du moins en partie, les inconvénients constatés dans les méthodes de l'art antérieur. En particulier, l'invention définit une méthode permettant une séparation et une identification des isoenzymes de l'ALP, améliorée sur le plan de la spécificité et de la sensibilité.

La présente invention met en outre à la disposition des utilisateurs et en particulier des cliniciens, un procédé de séparation, d'identification et de quantification des principales isophosphatases alcalines, réalisable en une seule étape, sur un support d'électrophorèse unique facile à produire.

L'invention a donc pour objet, un nouveau procédé de séparation et d'identification des isoenzymes de l'ALP, par électrophorèse, caractérisé en ce que l'on dépose la lectine, de façon localisée sur le support d'électrophorèse.

La lectine déposée en solution de façon localisée, peut diffuser dans le support, tout en restant localisée en une zone déterminée de ce support pendant la migration électrophorétique.

Le dépôt en question, localisé à proximité de la zone de dépôt de l'échantillon, se démarque du dépôt uniforme sur une zone étendue ou sur la totalité du support d'électrophorèse, décrit dans l'état de la technique.

L'invention , selon la revendication 1, a donc pour objet un procédé de séparation par électrophorèse, à partir d'un échantillon biologique, des isoenzymes de la phosphatase alcaline

L'interaction en question conduit à la formation d'un complexe entre la lectine et l'isoenzyme de l'ALP, jusqu'à l'obtention d'un équilibre.

Le procédé selon l'invention permet d'agir de façon beaucoup plus spécifique sur la fraction osseuse de l'ALP que sur les autres fractions de l'ALP, du fait de la réaction de cette fraction avec la lectine.

L'échantillon biologique analysé peut être tout échantillon biologique susceptible de contenir de l'ALP et en particulier, un échantillon de fluide biologique tel qu'un échantillon de sérum ou de plasma, ou éventuellement un extrait tissulaire, prélevé chez un patient.

Dans le cadre de l'invention , l'électrophorèse est réalisée sur tout support d'électrophorèse approprié, en particulier sur gel, notamment sur gel d'agarose ou de polyacrylamide, ou sur une membrane poreuse, en particulier d'acétate de cellulose.

Les conditions particulières définies ci-dessus pour la réalisation de l'électrophorèse selon l'invention, peuvent être appliquées dans le cadre des méthodes connues d'électrophorèse, qu'il s'agisse de méthodes automatisées ou non.

La zone du dépôt localisé de la lectine est déterminée en fonction du sens de la migration de l'échantillon et de la lectine. Ainsi, on choisira la zone du dépôt de la solution de lectine de façon telle que l'échantillon, lors de la migration, croise la lectine, la mobilité de cette dernière lors de la migration devant être prise en compte. De même on tiendra compte de la position finale normalement atteinte par les autres isoenzymes de l'ALP, pour déterminer la zone de dépôt de la lectine par rapport à celle de l'échantillon. En pratique, la lectine et l'échantillon sont éloignés de 1 à 10mm, avantageusement de 5mm, au moment du dépôt de l'échantillon.

Les autres conditions du dépôt localisé de lectine telles que la concentration de lectine, le temps d'application sur le support d'électrophorèse, sont déterminées de telle sorte qu'elles rendent possible la séparation des isoenzymes osseuse et hépatique de l'ALP lors de l'électrophorèse, dans les conditions de la réaction de migration.

En d'autres termes, le procédé de l'inventionpermet, une fois les paramètres de l'électrophorèse déterminés en ce qui concerne en particulier le dépôt de la lectine, la séparation des isoenzymes osseuse et hépatique, dans des conditions satisfaisantes pour les identifier par rapport aux autres isoenzymes de l'ALP et de préférence pour les quantifier. La mobilité des autres isoenzymes de l'ALP, affectée pendant le passage à travers la zone de présence de la lectine, redevient conforme à la normale en dehors de cette zone.

La fraction osseuse de l'ALP étant la plus sialilée, sa migration électrophorétique est la plus affectée par le passage de l'échantillon à travers la lectine déposée sur le support. En conséquence elle est précipitée en une zone pouvant être distinguée de la zone de migration des autres isoenzymes de l'ALP.

Selon l'invention, le procédé de séparation par électrophorèse des isoenzymes de la phosphatase alcaline (ALP), est caractérisé en ce qu'il comprend :

- le dépôt sur le support d'électrophorèse de l'échantillon biologique contenant les isoenzymes de l'ALP à séparer,
- le dépôt en une zone determine localisée sur le support d'électrophorèse, d'une solution de lectine capable d'interagir avec les isoenzymes de l'ALP contenues dans l'échantillon,
- l'application d'un champ électrique pour permettre la séparation électrophorétique, par migration des isoenzymes de l'ALP dans des conditions permettant la séparation différentielle des fractions osseuse et hépatique de l'ALP,
- la révélation des isoenzymes séparées de l'ALP.

La révélation est réalisée de toute façon en soi connue, de préférence à l'aide d'un substrat de l'ALP.

On peut, à l'issue des étapes aboutissant à la séparation sur le support d'électrophorèse des isoenzymes de l'ALP, procéder à une analyse quantitative des différentes isoenzymes séparées, ou de certaines d'entre elles.

Pour quantifier les isoenzymes de de l'ALP détectées à l'issue de la réalisation de l'électrophorèse, différentes méthodes peuvent utilisées. On aura par exemple recours à la mesure de la densitométrie du support d'électrophorèse, après coloration des fractions séparées de l'ALP au moyen d'un substrat de l'ALP.

Lorsque la fraction osseuse de l'ALP est en excès par rapport à la capacité de fixation de la lectine déposée sur le gel, la lectine peut ne pas précipiter cette fraction en totalité. Dans ce cas, la partie non précipitée continue de migrer avec les autres fractions, et se retrouve sous la forme d'une trainéeau voisinage des isoenzymes sous jacentes notammment H2, I1, I2 et I3. Pour quantifier les différentes isoenzymes on peut dans ce cas avoir recours à un deuxième dépôt du même échantillon sur le même support, en l'absence de lectine. Sur le profil sans lectine, on détermine les pourcentages du bloc H1 Os P1 et des fractions séparées H2, I1, P2, I2, I3 et UF. Le pourcentage de fraction Os est obtenu en retranchant au bloc H1 Os P1 les pourcentages H1 et P1 déterminés sur le profil avec lectine.

Dans le cadre des définitions données ci-dessus, de la mise en oeuvre de l'électrophorèse en présence d'un dépôt localisé de lectine sur le support d'électrophorèse, l'invention permet d'effectuer le dépôt de lectine et le dépôt de l'échantillon de façon simultanée sur le support d'électrophorèse.

Selon l'invention, le dépôt de l'échantillon et le dépôt de lectine peuvent être décalés dans le temps.

En outre les dépôts respectivement de l'échantillon et de la solution de lectine sur le support d'électrophorèse peuvent être réalisés pendant des durées identiques ou au contraire différentes.

Avantageusement dans le cadre des définitions données ci-dessus, le cas échéant prises en combinaison, la lectine et l'échantillon sont déposés pendant une durée sensiblement identique, et de préférence simultanément pour des raisons pratiques et économiques.

Pour un échantillon donné et une concentration de lectine dans la solution déterminée, on choisit la durée de l'application de l'échantillon et de la lectine et donc la quantité d'échantillon et de lectine déposés, sur le support d'électrophorèse de façon à obtenir la précipitation de la fraction Os des isoenzymes de l'ALP de telle sorte que les fractions osseuse et hépatique sont séparées lors de la migration. Pour la détermination de la concentration de la solution de lectine et de la durée d'application de la solution sur le support d'électrophorèse, on tient compte également de la température atteinte par le support au cours de la migration.

En effet, l'interaction de la lectine avec la fraction ALP osseuse étant dépendante de la température, il y a lieu de tenir compte de la température du support d'électrophorèse pendant l'étape de migration. Une diminution de l'interaction lectine - ALP osseuse due à une augmentation de la température du support d'électrophorèse, peut être compensée par une augmentation de la quantité de lectine déposée et donc par exemple en augmentant la concentration de la solution de lectine utilisée.

Ces paramètres peuvent être déterminés à la lumière des indications qui suivent et des valeurs données à titre d'exemples et si besoin adaptés à des conditions choisies d'électrophorèse, en réalisant des tests tels que ceux qui figurent dans les exemples donnés ci-après.

La durée d'application de l'échantillon et ou de la solution de lectine peut varier et notamment être comprise entre 5 et 20 min, de préférence 15 minutes, pour l'échantillon et/ou pour la lectine.

Ce dépôt peut être effectué par tous moyens connus manuels ou automatisés par exemple au moyen d'applicateurs de type « peignes », par exemple des dispositifs décrits dans la demande de brevet européen n°0493996.

La lectine utilisée est sous la forme d'une solution aqueuse.

Ainsi, la concentration de lectine déposée sur le support d'électrophorèse utilisé dans des conditions habituelles est comprise entre 0,1 mg/ml et la limite de solubilité de la lectine dans l'eau. Cette concentration est avantageusement entre 0,5 et 15 mg/ml, de préférence entre 1 et 10 mg/ml.

Selon un mode de réalisation particulier de l'invention, le procédé est caractérisé en ce que lorsque la concentration en lectine est comprise entre 1 et 10 mg/ml, la température de migration est comprise entre respectivement 18°C et 53°C. Ces conditions sont notamment applicables pour un dépôt de la solution de lectine pendant une durée voisine d'environ 15 minutes avec un applicateur du type peigne tel que décrit dans la demande de brevet EP 0493996.

L'invention concerne en particulier un mode de réalisation du procédé d'électrophorèse, selon lequel, lorsque les isoenzymes de l'ALP présentes dans l'échantillon testé présentent une mobilité électrophorétique en direction de l'anode, la lectine est déposée entre l'anode et la zone de dépôt de l'échantillon.

Avantageusement dans ce cas, la lectine est déposée entre le point de dépôt de l'échantillon biologique et la zone normalement occupée par la fraction intestinale 3(13) à l'issue de l'étape de migration, lorsque cette fraction est présente dans l'échantillon.

Différentes lectines peuvent être mises en oeuvre dans le cadre de l'invention. Les lectines sont des protéines fixant les groupements sialilés. Parmi les lectines utilisables dans le cadre de l'invention, on citera la lectine de germe de blé (Triticum vulgaris) ou WGA (Wheat Germ Agglutinin). La lectine WGA peut être obtenue auprès des sociétés Sigma, Pharmacia...

L'invention a également pour objet, un procédé répondant aux définitions données ci-dessus, prises séparément ou en combinaison, dans lequel la séparation des fractions autres que osseuse et hépatique 1 de l'ALP est améliorée.

L'invention met donc à disposition, dans des conditions compatibles avec les analyses faites en routine en laboratoire, des moyens permettant la détection *in vitro* dans un échantillon biologique, de la présence anormale d'une ou plusieurs isoenzymes de l'ALP.

Ce procédé peut en particulier être mis en oeuvre dans le cadre d'un protocole pour le diagnostic d'une pathologie hépatique ou biliaire correspondant à une présence anormale de la fraction hépatique de l'ALP. L'invention permet aussi la recherche de la présence anormale de la fraction osseuse dans le cadre du diagnostic de pathologies osseuses.

Entrent également dans le cadre de l'invention, des kits pour la réalisation de la séparation par électrophorèse, des fractions constituées par les isoenzymes de l'ALP.

Le procédé de l'invention offre donc l'avantage de permettre, sur un support unique d'électrophorèse, sans traitement préalable de réchantillon, la détermination qualitative de toutes les isoenzymes de l'ALP en un dépôt et leur détermination quantitative en deux dépôts sur un support unique. Le support d'électrophorèse est dépourvu de lectine avant son utilisation, ce qui permet de le conserver dans des conditions de température habituelle et ne modifie pas son prix de revient.

L'invention a donc pour objet l'utilisation d'une solution de lectine pour la réalisation d'un dépôt en une zone localisée sur un support d'électrophorèse.

La demande décrit aussi un kit comprenant par exemple :

-   un support d'électrophorèse comprenant un matériau poreux approprié pour le dépôt d'un échantillon biologique à analyser et pour la réalisation de la migration électrophorétique,
-   une solution de lectine à une concentration comprise entre 0,1 mg/ml et 15 mg/ml, avantageusement entre 0,1 et 15 mg/ml de préférence entre 1 et 10mg/ml.

Le kit décrit dans la présente demande peut en outre comprendre le ou les tampons nécessaires à la réaction d'électrophorèse. Il peut aussi contenir les réactifs de révélation de l'activité de l'ALP.

D'autres caractéristiques et avantages de l'invention apparaissent dans les exemples qui suivent.

EXEMPLES

Principes de la réaction mettant en oeuvre la lectine lorsqu'elle est uniformément répartie dans le gel d'électrophorèse.

L'interaction de la lectine WGA avec une isophosphatase peu sialilée peut être représentée par l'équilibre :

(A) Isophosphatase +WGA $\rightleftarrows$ Complexe {Isophosphatase-WGA}

Dans les conditions d'électrophorèse utilisées normalement, soit à pH basique, les isophosphatases ont une mobilité anodique alors que la lectine a une mobilité légèrement cathodique. Le complexe {Isophosphatase-WGA} aura donc une mobilité anodique plus faible que l'isophosphatase libre.

Durant toute la durée de la migration, l'isophosphatase va interagir avec la lectine WGA selon l'équilibre de la réaction (A), et sera donc ralentie.

Ainsi, le profil obtenu sur un gel sans lectine WGA où, dans des conditions déterminées, toutes les fractions étaient séparées à l'exception du bloc constitué par les isoenzymes H1, Os (éventuellement P1), va conduire sur le gel incorporant sur toute sa surface de la lectine WGA, à un profil où la H1 (éventuellement P1) sera dégagée de la osseuse mais où les fractions H1, P1 d'une part, et H2, 12 d'autre part, qui étaient résolues sur le gel sans lectine WGA, seront confondues. Il serait donc nécessaire, même pour une simple estimation qualitative des isoenzymes d'un échantillon, d'avoir recours à son électrophorèse sur les 2 types de gels, ce qui alourdit d'autant l'analyse.

Outre cet inconvénient, la lectine WGA est thermo-sensible ce qui rend la production des gels la contenant très délicate ; de plus, les utilisateurs ont l'obligation de conserver ces gels entre 4 et 8°C afin de préserver intactes leurs performances.

Principes de la réaction selon l'invention, mettant en oeuvre la lectine déposée de façon localisée sur le gel d'électrophorèse

Dans le cadre de l'invention, une solution de lectine WGA est déposée en avant du dépôt de l'échantillon, c'est à dire entre l'échantillon et l'anode. Le gel d'électrophorèse n'est donc pas imprégné de la lectine sur toute sa surface. La lectine WGA est déposée simultanément ou non à l'échantillon. Les deux dépôts étant réalisés, la tension est appliquée pour obtenir la séparation électrophorétique.

Dans ces conditions, la plus grande partie de la fraction Os précipite quand elle traverse la zone où a été déposée la lectine WGA. La mobilité électrophorétique des autres fractions isophosphatases est affectée par la lectine WGA selon l'équilibre de la réaction :

$$\text{Isophosphatase} + \text{WGA} \rightleftarrows \text{Complexe } \{\text{isophosphatase-WGA}\},$$

mais uniquement lors de la traversée de la zone contenant la lectine WGA. Cette zone est très réduite (<1 mm) ce qui permet de conserver un profil pratiquement identique à celui obtenu en l'absence de lectine, à l'exception de la fraction osseuse qui se trouve dégagée des fractions H1 et P1. Dans ces conditions, l'électrophorèse de l'échantillon avec dépôt de lectine en avant de l'échantillon permet l'analyse qualitative sur un seul gel et en une seule étape : l'identification de toutes les isoenzymes d'après leur position est accessible et sans qu'il soit nécessaire de procéder à des traitements complémentaires de l'échantillon.

Dans les cas où il est nécessaire de quantifier les différentes isoenzymes qui ont été séparées et révélées par un réactif approprié, il y a lieu de tenir compte du fait que la précipitation de la fraction osseuse par la lectine WGA n'est pas totale, en particulier dans le cas où cette fraction osseuse est fortement augmentée. Une partie des molécules de phosphatase alcaline osseuse échappe à la précipitation lors de la traversée du dépôt de lectine. La liaison Os-WGA est cependant suffisante pour que ces molécules de phosphatase alcaline osseuse non précipitées entraînent avec elles de la lectine WGA. Elles vont donc être suffisamment freinées pour ne pas atteindre la zone H1P1. On obtient alors, en plus de l'arc de précipitation de la fraction osseuse, une traînée de phosphatase alcaline osseuse qui atteint la zone où migre la H2. Dans ces conditions, la quantification des isoenzymes sous jacentes à cette traînée, soit les isoenzymes H2, I1, 12,13 est perturbée par cette traînée.

Pour effectuer la quantification dans une situation où la quantité de la fraction Os risque d'être supérieure à ce qui peut précipiter, il y a donc lieu de réaliser côte à côte deux dépôts du même échantillon, un avec dépôt de lectine, l'autre sans. Sur le profil avec lectine WGA les fractions H1 et P1 peuvent être quantifiées. Sur le profil sans lectine on détermine les pourcentages du bloc H1 Os P1 et des fractions séparées H2, I1, P2, I2, I3 et UF. Le pourcentage de fraction Os est obtenu en retranchant au bloc H1 Os P1 les pourcentages H1 et P1 déterminés sur le profil avec lectine.

On peut donc déterminer sur un seul gel, en une seule étape avec deux dépôts, le pourcentage de toutes les isoenzymes sans aucun traitement préalable de l'échantillon.

Concentration de lectine et température de l'électrophorèse

La concentration de lectine à utiliser et la température de migration (température du support d'électrophorèse) sont étroitement liées. En effet, l'équilibre de formation du complexe isophosphatase-lectine WGA est dépendant de la température. Ainsi, la dissociation du complexe est favorisée par l'augmentation de température. La concentration de lectine devra être augmentée pour obtenir le même pouvoir précipitant de la fraction osseuse.

Dans la pratique, la concentration de lectine à utiliser est fonction de la température du gel et peut être choisie conformément au schéma suivant :

| Température du gel pendant la migration °C | Concentration de lectine mg/ml |
|---|---|
| 18 | 1 |
| 28 | 2 |
| 38 | 3 |

(suite)

| Température du gel pendant la migration °C | Concentration de lectine mg/ml |
|---|---|
| 48 | 5 |
| 53 | 10 |

Donc, si le procédé est mis en oeuvre selon l'invention avec un système d'électrophorèse qui ne permet pas de contrôler la température de migration (exemple 1), la concentration de lectine doit être augmentée pour tenir compte de la température maximale atteinte par le gel durant la migration. Cette température dépend d'un certain nombre de paramètres comme la force ionique et les dimensions du gel, les paramètres de migration et la température extérieure au cours de la migration. Dans les conditions utilisées dans l'exemple 1, la température maximale atteinte par le gel est voisine de 38°C, une concentration de lectine de 3 mg/ml est donc utilisée. Dans l'exemple 2, l'instrument permet de réguler la température de l'électrophorèse à 20°C. Cependant, pour une électrophorèse qui se déroule à la puissance constante de 20 W, la température effective du gel est de 28°C. Par conséquent, la concentration de lectine nécessaire pour obtenir l'effet désiré est de 2 mg/ml.

**Position et durée du dépôt de lectine**

Dans les conditions de pH (basique) utilisées pour l'électrophorèse les isophosphatases alcalines présentent toutes une mobilité en direction de l'anode, la lectine présente en revanche une très faible mobilité en direction de la cathode. Pour que la fraction osseuse rencontre la lectine, la lectine doit impérativement être déposée entre l'échantillon et l'anode. Plus précisément, elle doit être déposée à une distance inférieure ou égale à celle parcourue par la fraction osseuse dans les conditions de migration utilisées et en l'absence de lectine. Cependant, afin d'éviter de précipiter la fraction osseuse dans une zone déjà occupée par d'autres fractions, il apparaît plus judicieux de déposer la lectine à une distance comprise entre l'échantillon et la position qu'occupe la fraction 13 en fin de migration. En pratique, la lectine est déposée devant l'échantillon à une distance comprise entre 1 et 10 mm et de préférence vers 5 mm.

La lectine est déposée en même temps ou après l'échantillon. La simultanéité des 2 dépôts n'est pas obligatoire. Cependant, si l'échantillon et la lectine ne sont pas déposés en même temps, il faut veiller à éviter la diffusion du premier dépôt pendant la durée du second. C'est pourquoi il est aisé de déposer l'échantillon et la lectine simultanément et pendant la même durée. De plus, les deux dépôts sont parallèles entre eux et perpendiculaires au sens de migration.

**Exemple de réalisation N°1**

Gel de composition suivante :

| | |
|---|---|
| Agarose | 1% |
| Tris | 0,03 M |
| Véronal sodé | 0,025 M |
| Véronal acide | 0,005 M |
| Azoture de sodium | 1 g/l |
| Triton X100 | 10 g/l |
| Nonidet NP 40 | 5 g/l. |

Dans un erlenmeyer de 100 ml sont introduits 40 ml d'eau déminéralisée et 0,5 g d'agarose. Après 5 minutes d'ébullition sous agitation constante, l'agarose est dissous et donne une solution parfaitement limpide. La température de cette solution est ramenée à 50 °C dans un bain thermostaté. Dans un erlenmeyer de 50 ml, sont introduits 10 ml de tampon concentré en solution contenant 18 g/l de Tris, 27,75 g/l de véronal sodé, 4,6 g/l de véronal acide, 5 g/l d'azoture de sodium, 50 g/l de Triton X100 et 25 g/l de Nonidet NP 40. Cette solution est maintenue à 50°C dans le bain thermostaté.

Le tampon ainsi préchauffé est ajouté à la solution d'agarose. L'ensemble est homogénéisé et maintenu à 50°C.

5 ml de la solution précédente prélevée au moyen d'une pipette sont alors coulés uniformément sur un film de plastique hydrophile de 10 x 8 cm.

Après gélification et stabilisation, le gel peut être utilisé. Les sérums frais à analyser sont déposés en 2 dépôts adjacents, à 2,5 cm du bord, du côté de la cathode. Une solution à 3 mg/ml de lectine Wheat Germ Agglutinin (WGA) est appliquée, simultanément et durant le même temps, à 32 mm devant l'un des 2 dépôts de chaque échantillon, c'est à dire entre l'échantillon et l'anode. Le temps d'application peut être de 15 minutes pour chaque dépôt réalisé avec un applicateur à membrane microporeuse tel que commercialisé par Sebia et décrit dans la demande EP0493996.

La séparation des isophosphatases alcalines est obtenue par électrophorèse dans une cuve dont les bacs contiennent un tampon Tris 0,003 M véronal sodé 0.025 M,véronal acide 0,005 M, azoture de sodium 0,1 g/l pendant une durée de 50 minutes sous une tension de 100 V constants.

Les activités phosphatases alcalines sont alors révélées en incubant le gel avec un substrat classique de cette enzyme (par exemple BromoChloroIndolyl phosphate et nitrobluetetrazolium). Une coloration bleue est alors obtenue à l'emplacement de chaque fraction isophosphatase, proportionnelle à son activité. Après révélation, le gel est lavé puis séché et analysé par densitométrie afin de quantifier les différentes isophosphatases alcalines.

## Exemple de réalisation N°2

Gel de composition suivante :

| | |
|---|---|
| Agarose | 1% |
| Tris | 0,38 M |
| Acide borique | 0,06 M |
| Azoture de sodium | 1 g/l |
| Triton X100 | 10 g/l |
| Nonidet NP 40 | 5 g/l |

Dans un erlenmeyer de 100 ml sont introduits 40 ml d'eau déminéralisée et 0,5 g d'agarose. Après 5 minutes d'ébullition sous agitation constante, l'agarose est dissous et donne une solution parfaitement limpide. La température de cette solution est ramenée à 50°C dans un bain thermostaté. Dans un erlenmeyer de 50 ml, sont introduits 10 ml de tampon concentré en solution contenant 229,9 g/l de Tris, 18,55 g/l d'acide borique, 5 g/l d'azoture de sodium, 50 g/l de Triton X100 et 25 g/l de Nonidet NP 40. Cette solution est maintenue à 50 °C dans le bain thermostaté.

Le tampon ainsi préchauffé est ajouté à la solution d'agarose. L'ensemble est homogénéisé et maintenu à 50°C.

5 ml de la solution précédente prélevée au moyen d'une pipette sont alors coulés uniformément sur un film de plastique hydrophile de 10 x 8 cm.

Après gélification et stabilisation, le gel peut être utilisé. Les sérums frais à analyser sont déposés en 2 dépôts adjacents, à 2,5 cm du bord, du côté de la cathode. Une solution à 2 mg/ml de lectine Wheat Germ Agglutinin (WGA) est appliquée, simultanément et durant le même temps, à 5 mm devant l'un des deux dépôts de chaque échantillon, c'est à dire entre l'échantillon et l'anode.

La séparation des isophosphatases alcalines est obtenue par électrophorèse dans un instrument permettant de réguler la température à 20°C. La migration est réalisée à une puissance constante de 20 W et pendant 20 minutes.

Le développement des activités phosphatases alcalines et la densitométrie se font comme dans l'exemple précédent.

## Revendications

1. Procédé de séparation par électrophorèse, à partir d'un échantillon biologique, des isoenzymes de la phosphatase alcaline (ALP), **caractérisé en ce qu'**il comprend :

   - le dépôt sur un support d'électrophorèse de l'échantillon biologique contenant les isoenzymes de l'ALP à séparer,
   - le dépôt en une zone déterminée localisée du support d'électrophorèse, d'une solution de lectine capable d'interagir avec les isoenzymes de l'ALP contenues dans l'échantillon,
   - l'application d'un champ électrique pour permettre l'interaction entre ladite lectine et les isoenzymes de l'ALP contenues dans l'échantillon biologique analysé, et la séparation électrophorétique, par migration des isoenzymes de l'ALP dans des conditions permettant la séparation différentielle des fractions osseuse et hépatique de l'ALP,
   - la révélation des isoenzymes séparées de l'ALP.

2. Procédé de séparation par électrophorèse selon la revendication 1, **caractérisé en ce que** la séparation électrophorétique des isoenzymes de l'ALP est suivie d'une étape d'analyse quantitative des différentes isoenzymes de l'ALP séparées.

3. Procédé de séparation par électrophorèse selon l'une quelconque des revendications 1 à 2, **caractérisé en ce que** le dépôt de la solution de lectine et le dépôt de l'échantillon biologique sont effectués simultanément.

4. Procédé de séparation par électrophorèse selon l'une quelconque des revendications 1 à 2, **caractérisé en ce que** le dépôt de la solution de lectine est réalisé postérieurement au dépôt de l'échantillon biologique.

5. Procédé de séparation par électrophorèse selon l'une quelconque des revendications 1 à 2, **caractérisé en ce que** le dépôt de la solution de lectine est réalisé antérieurement au dépôt de l'échantillon biologique

6. Procédé de séparation par électrophorèse selon l'une quelconque des revendications 1 à 4, **caractérisé en ce que** la durée du dépôt de la solution de lectine et/ou la durée du dépôt de l'échantillon, sont comprises entre 5 et 20 minutes.

7. Procédé de séparation par électrophorèse selon les revendications 1 à 6, **caractérisé en ce que** la concentration de la solution de lectine est déterminée en fonction de la température du support d'électrophorèse et de la durée de l'application de la solution de lectine sur le support.

8. Procédé de séparation par électrophorèse selon les revendications 1 à 7, **caractérisé en ce que** la solution de lectine a une concentration comprise entre 0,1 mg/ml et sa limite de solubilité dans l'eau.

9. Procédé de séparation par électrophorèse selon les revendications 1 à 7, **caractérisé en ce que** la solution de lectine a une concentration comprise entre 1 et 10 mg/ml.

10. Procédé de séparation par électrophorèse selon la revendication 9, **caractérisé en ce que** lorsque la concentration de la solution de lectine est comprise entre 1 et 10 mg/ml, la température de migration est comprise entre respectivement 18°C et 53°C.

11. Procédé de séparation par électrophorèse selon l'une quelconque des revendications 1 à 10, **caractérisé en ce que** la concentration de la solution de lectine est déterminée en fonction de la température maximale atteinte au sein du support d'électrophorèse lors de l'étape de migration.

12. Procédé de séparation par électrophorèse selon les revendications 1 à 11, **caractérisé en ce que**, lorsque les isoenzymes de l'ALP présentent une mobilité en direction de l'anode, la lectine est déposée, entre l'anode et l'échantillon biologique.

13. Procédé de séparation par électrophorèse selon la revendication 12, **caractérisé en ce que** la lectine est déposée entre l'échantillon biologique et la zone normalement occupée par la fraction intestinale 3(13) à l'issue de l'étape de migration, lorsque cette fraction est présente dans l'échantillon.

14. Procédé de séparation par électrophorèse selon l'une quelconque des revendications 1 à 13, **caractérisé en ce que** la lectine est la lectine de germes de blé (Wheat Germ Aglutinin ou WGA).

15. Procédé de séparation par électrophorèse selon les revendications 1 à 14, **caractérisé en ce que** le support d'électrophorèse est un gel d'agarose ou de polyacrylamide.

16. Procédé de séparation par électrophorèse selon les revendications 1 à 14, **caractérisé en ce que** le support d'électrophorèse est une membrane d'acétate de cellulose.

17. Procédé de séparation par électrophorèse selon les revendications 1 à 16, **caractérisé en ce que** les fractions de l'ALP révélées sont quantifiées.

18. Procédé de séparation par électrophorèse selon la revendication 17, **caractérisé en ce que** la quantification est réalisée par mesure de la densitométrie du support d'électophorèse, après coloration des fractions de l'ALP au moyen d'un substrat de l'ALP.

19. Procédé de séparation par électrophorèse selon les revendications 1 à 18, pour la détection de la présence anormale d'une ou plusieurs isoenzymes de l'ALP.

20. Procédé de séparation par électrophorèse selon les revendications 1 à 19, pour le diagnostic in vitro d'une pathologie hépatique ou biliaire.

21. Procédé de séparation par électrophorèse selon les revendications 1 à 19, pour le diagnostic in vitro d'une pathologie osseuse.

**Patentansprüche**

1. Verfahren zur Trennung von Isoenzymen der alkalischen Phosphatase (ALP), ausgehend von einer biologischen Probe, durch Elektrophorese, **dadurch gekennzeichnet, dass** es umfasst:

   - Auftragen der biologischen Probe, die die zu trennenden ALP-Isoenzyme enthält, auf einen Elektrophoreseträger,
   - Auftragen einer Lektinlösung, die zur Wechselwirkung mit den in der Probe enthaltenen ALP-Isoenzymen geeignet ist, in einer bestimmten lokalisierten Zone des Elektrophoreseträgers,
   - Anlegen eines elektrischen Feldes, um die Wechselwirkung zwischen dem Lektin und den in der analysierten biologischen Probe enthaltenen ALP-Isoenzymen und die elektrophoretische Trennung durch Migration von ALP-Isoenzymen unter Bedingungen zu ermöglichen, die die differenzierte Auftrennung von Knochen-ALP-Fraktionen und Leber-ALP-Fraktionen erlauben,
   - Nachweis der aufgetrennten ALP-Isoenzyme.

2. Verfahren zur Elektrophorese-Trennung nach Anspruch 1, **dadurch gekennzeichnet, dass** die elektrophoretische Trennung der ALP-Isoenzyme von einem quantitativen Analyseschritt der verschiedenen aufgetrennten ALP-Isoenzyme gefolgt ist.

3. Verfahren zur Elektrophorese-Trennung nach einem der Ansprüche 1 bis 2, **dadurch gekennzeichnet, dass** das Auftragen der Lektinlösung und das Auftragen der biologischen Probe gleichzeitig erfolgt.

4. Verfahren zur Elektrophorese-Trennung nach einem der Ansprüche 1 bis 2, **dadurch gekennzeichnet, dass** das Auftragen der Lektinlösung nach einem Auftragen der biologischen Probe erfolgt.

5. Verfahren zur Elektrophorese-Trennung nach einem der Ansprüche 1 bis 2, **dadurch gekennzeichnet, dass** das Auftragen der Lektinlösung vor einem Auftragen der biologischen Probe erfolgt.

6. Verfahren zur Elektrophorese-Trennung nach einem der Ansprüche 1 bis 4, **dadurch gekennzeichnet, dass** die Dauer des Auftragens der Lektinlösung und/oder die Dauer des Auftragens der Probe zwischen 5 und 20 Minuten liegt.

7. Verfahren zur Elektrophorese-Trennung nach einem der Ansprüche 1 bis 6, **dadurch gekennzeichnet, dass** die Konzentration der Lektinlösung in Funktion der Temperatur des Elektrophoreseträgers und der Dauer des Aufbringens der Lektinlösung auf den Träger bestimmt wird.

8. Verfahren zur Elektrophorese-Trennung nach einem der Ansprüche 1 bis 7, **dadurch gekennzeichnet, dass** die Lektinlösung eine Konzentration zwischen 0,1 mg/ml und ihrer Löslichkeitsgrenze in Wasser aufweist.

9. Verfahren zur Elektrophorese-Trennung nach einem der Ansprüche 1 bis 7, **dadurch gekennzeichnet, dass** die Lektinlösung eine Konzentration zwischen 1 und 10 mg/ml aufweist.

10. Verfahren zur Elektrophorese-Trennung nach Anspruch 9, **dadurch gekennzeichnet, dass**, wenn die Konzentration der Lektinlösung zwischen 1 und 10 mg/ml liegt, die Migrationstemperatur entsprechend zwischen 18 °C und 53 °C liegt.

11. Verfahren zur Elektrophorese-Trennung nach einem der Ansprüche 1 bis 10, **dadurch gekennzeichnet, dass** die Konzentration der Lektinlösung in Funktion der beim Migrationsschritt im Inneren des Elektrophoreseträgers erreichten maximalen Temperatur bestimmt wird.

12. Verfahren zur Elektrophorese-Trennung nach einem der Ansprüche 1 bis 11, **dadurch gekennzeichnet, dass**, wenn die ALP-Isoenzyme eine Mobilität in Richtung der Anode aufweisen, das Lektin zwischen der Anode und der biologischen Probe abgesetzt wird.

13. Verfahren zur Elektrophorese-Trennung nach Anspruch 12, **dadurch gekennzeichnet, dass** das Lektin zwischen der biologischen Probe und der am Ende des Migrationsschritts normalerweise von der Darm-Fraktion 3(13) eingenommenen Zone abgesetzt wird, wenn diese Fraktion in der Probe vorhanden ist.

14. Verfahren zur Elektrophorese-Trennung nach einem der Ansprüche 1 bis 13, **dadurch gekennzeichnet, dass** das Lektin Weizenkeimlektin ist (Wheat Germ Aglutinin oder WGA).

15. Verfahren zur Elektrophorese-Trennung nach einem der Ansprüche 1 bis 14, **dadurch gekennzeichnet, dass** der Elektrophoreseträger ein Agarosegel oder Polyacrylamidgel ist.

16. Verfahren zur Elektrophorese-Trennung nach einem der Ansprüche 1 bis 14, **dadurch gekennzeichnet, dass** der Elektrophoreseträger eine Celluloseacetatmembran ist.

17. Verfahren zur Elektrophorese-Trennung nach einem der Ansprüche 1 bis 16, **dadurch gekennzeichnet, dass** die nachgewiesenen ALP-Fraktionen quantitativ analysiert werden.

18. Verfahren zur Elektrophorese-Trennung nach Anspruch 17, **dadurch gekennzeichnet, dass** die quantitative Analyse durch Densitometrie des Elektrophoreseträgers nach Anfärben der ALP-Fraktionen mittels eines ALP-Substrats durchgeführt wird.

19. Verfahren zur Elektrophorese-Trennung nach einem der Ansprüche 1 bis 18, zum Nachweis eines anormalen Vorliegens von einem oder mehreren ALP-Isoenzymen.

20. Verfahren zur Elektrophorese-Trennung nach einem der Ansprüche 1 bis 19, zur Diagnose eines pathologischen Zustands von Leber oder Galle *in vitro.*

21. Verfahren zur Elektrophorese-Trennung nach einem der Ansprüche 1 bis 19, zur Diagnose eines pathologischen Zustands von Knochen *in vitro.*

**Claims**

1. A process for separating alkaline phosphatase (ALP) isoenzymes from a biological sample by electrophoresis, **characterized in that** it comprises:

   ● depositing the biological sample containing the ALP isoenzymes to be separated on an electrophoresis support;
   ● depositing a lectin solution that is capable of interacting with the ALP isoenzymes contained in the sample on a predetermined localized zone of the electrophoresis support;
   ● applying an electric field to permit said lectin to interact with the ALP isoenzymes contained in the analyzed biological sample and to permit electrophoretic separation by migration of the ALP isoenzymes under conditions which can permit differential separation of the osseous and hepatic ALP fractions;
   ● revealing the separated ALP isoenzymes.

2. A process for separation by electrophoresis according to claim 1, **characterized in that** the electrophoretic separation of the ALP isoenzymes is followed by a step for quantitative analysis of the various separated ALP isoenzymes.

3. A process for separation by electrophoresis according to claim 1 or claim 2, **characterized in that** the lectin solution and the biological sample are deposited simultaneously.

4. A process for separation by electrophoresis according to claim 1 or claim 2, **characterized in that** the lectin solution is deposited after the biological sample has been deposited.

5. A process for separation by electrophoresis according to claim 1 or claim 2, **characterized in that** the lectin solution is deposited before the biological sample is deposited.

6. A process for separation by electrophoresis according to any one of claims 1 to 4, **characterized in that** the period for depositing the lectin solution and/or the period for depositing the sample is/are in the range 5 to 20 minutes.

7. A process for separation by electrophoresis according to any one of claims 1 to 6, **characterized in that** the concentration of the lectin solution is determined as a function of the temperature of the electrophoresis support and the period over which the lectin solution is applied to the support.

8. A process for separation by electrophoresis according to any one of claims 1 to 7, **characterized in that** the concentration of the lectin solution is in the range 0.1 mg/ml to its limit of solubility in water.

9. A process for separation by electrophoresis according to any one of claims 1 to 7, **characterized in that** the concentration of the lectin solution is in the range 1 to 10 mg/ml.

10. A process for separation by electrophoresis according to claim 9, **characterized in that** when the concentration of the lectin solution is in the range 1 to 10 mg/ml, the migration temperature is in the range 18°C to 53°C respectively.

11. A process for separation by electrophoresis according to any one of claims 1 to 10, **characterized in that** the concentration of the lectin solution is determined as a function of the maximum temperature reached in the electrophoresis support during the migration step.

12. A process for separation by electrophoresis according to any one of claims 1 to 11, **characterized in that** when the mobility of the ALP isoenzymes is in the anode direction, the lectin is deposited between the anode and the biological sample.

13. A process for separation by electrophoresis according to claim 12, **characterized in that** the lectin is deposited between the biological sample and the zone normally occupied by the intestinal fraction 3 (I3) at the end of the migration step, when this fraction is present in the sample.

14. A process for separation by electrophoresis according to any one of claims 1 to 13, **characterized in that** the lectin is wheat germ lectin (wheat germ agglutinin or WGA).

15. A process for separation by electrophoresis according to claims 1 to 14, **characterized in that** the electrophoresis support is an agarose gel or polyacrylamide.

16. A process for separation by electrophoresis according to claims 1 to 14, **characterized in that** the electrophoresis support is a cellulose acetate membrane.

17. A process for separation by electrophoresis according to claims 1 to 16, **characterized in that** the ALP fractions that are revealed are quantified.

18. A process for separation by electrophoresis according to claim 17, **characterized in that** the quantification is carried out by measuring the densitometry of the electrophoresis support after staining the ALP fractions using an ALP substrate.

19. A process for separation by electrophoresis according to claims 1 to 18, for detecting the abnormal presence of one or more ALP isoenzymes.

20. A process for separation by electrophoresis according to claims 1 to 19, for *in vitro* diagnosis of a hepatic or biliary disorder.

21. A process for separation by electrophoresis according to claims 1 to 19, for *in vitro* diagnosis of an osseous disorder.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5264098 A
- EP 0131606 A
- US 5667654 A
- EP 0493996 A

**Littérature non-brevet citée dans la description**

- **VAN HOOF V.O. ; DE BROE MARC, E.** *Clinical Laboratory Sciences,* 1994, vol. 31 (3
- **SIDNEY B. ROSALSKI ; A. YING FOO.** *Clinical Chemistry,* 1984, vol. 30/7, 1182-1186